# EUROPEAN PATENT APPLICATION

(11) **EP 0 976 714 A1**
(43) Date of publication of application: **02.02.2000**
(21) Application number: 98305368.7
(22) Date of filing: 06.07.1998
(51) Int. Cl.: C07C 67/343, C07C 45/69

(54) **Addition of ketones to alkenes**

(71) Applicant: QUEST INTERNATIONAL B.V., 1411 GP Naarden (NL)
(72) Inventor: The designation of the inventor has not yet been filed
(74) Representative: Humphries, Martyn

(57) **Abstract**

The invention provides radical addition of ketone to alkene of the form: 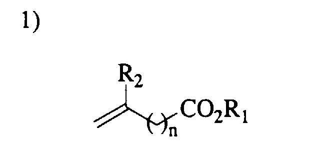 where
R₁ = alkyl
R₂ = H or alkyl
n = 1 to 8
or 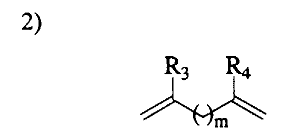 where
R₃ = H or lower alkyl
R₄ = H or lower alkyl
m = 2 to 8.

The process can be used to produce precursors useful in the production of macrocyclic ketones, particularly muscone.

## Description

### Field of the Invention

This invention concerns addition of ketones to alkenes.

### Summary of the Invention

In one aspect the invention provides radical addition of ketone to alkene of the form: 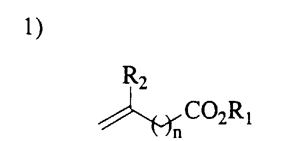 where
- R₁ =: alkyl
- R₂ =: H or alkyl
- n =: 1 to 8
or 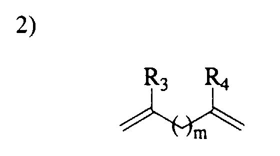 where
- R₃ =: H or lower alkyl
- R₄ =: H or lower alkyl
- m =: 2 to 8.
R₁ and R₂ are preferably lower alkyl groups.

The term "lower alkyl" is used in this specification to mean an alkyl group having from 1 to 4 carbon atoms.

The ketone has the general formula: 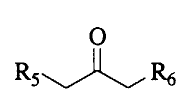 where
- R₅ =: H or lower alkyl
- R₆ =: H or lower alkyl

Suitable ketones include, for example, acetone, 2-butanone, 3-pentanone etc.

For alkenes of the form 1) the addition reaction is as follows:

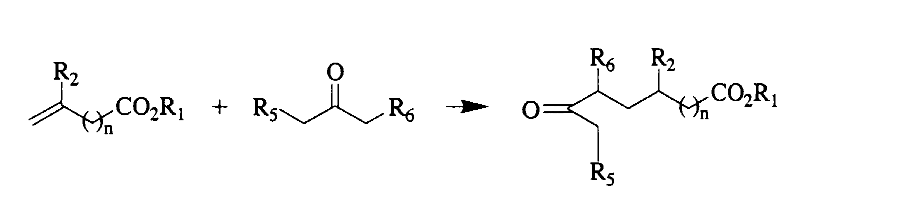

This reaction will be referred to herein as scheme 1.

For alkenes of the form 2) the addition reaction is as follows:

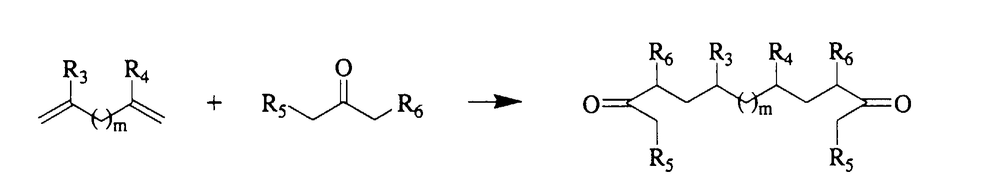

This reaction will be referred to herein as scheme 2.

Initiation of the addition reaction is preferably carried out by *in situ* generation of manganese (III) from manganese (II) by redox reaction, eg mediated by potassium permanganate, as this gives good chemoselectivity (see T. Linker *et al, Tetrahedron,* **51**, 9917, (1995)). Manganese (III) may alternatively be generated *in situ* from manganese (II) by anodic oxidation (eg as described in I. Nishiguchi *et al., Tetrahedron,* **47**, 831 (1991) and R. Warinsky, E. Steckham, *J Chem. Soc. Perkin 1*, 2007, (1994)). A further possibility is direct use of manganese (III) acetate, although this is less favoured because of difficulties of preventing over-oxidation of the intermediate radical educts.

Alkene starting materials can be obtained commercially or can be synthesised, eg by Wittig reaction.

A wide range of products can be made by the process of the invention, determined by the ketone and alkene selection for reaction. At least some of these products are useful as intermediates in production of macrocyclic ketones, including muscone, with useful fragrance properties. For the reaction products of the process of the invention to be capable of forming a macrocyclic ketone, there need to be sufficient carbon atoms between the keto- and ester functionalities for scheme 1 and the two keto-functionalities for scheme 2. For these purposes it is therefore preferred to use alkenes of form 1) in which n=8 (ie undecylenic acid esters) or alkenes of form 2) in which m=5,6,7 or 8.

Further, if the ultimate product has too high a molecular weight (eg being produced from a long chain ester and a larger ketone) it will be outside the range of olfaction. Smaller ketones, particularly acetone and 2-butanone, are therefore generally favoured.

Muscone is the common name of (-)-3-methylcyclopentadecanone which has the following structure:

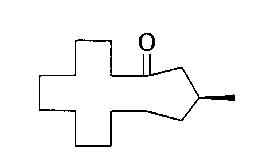

Muscone is the principal odorous constituent of the musk deer gland, and is a useful fragrance material. Since elucidation of its structure in 1926 (see Ruzicka, L., *Helv. Chim. Acta,* **9**, 230 (1926)), many syntheses both of the optically-active material and racemic muscone have been reported. These syntheses, while elegant, generally involve many stages and may require expensive and/or inaccessible starting materials. Hitherto there has thus been no short and efficient method of producing muscone which utilises inexpensive and readily accessible starting materials.

In accordance with the invention, for reaction scheme 1, reaction of methyl undecylenate (R₁=CH₃, R₂=H, n=8)(or other ester of undecylenic acid) with acetone produces methyl 13-oxotetradecanoate, as follows:

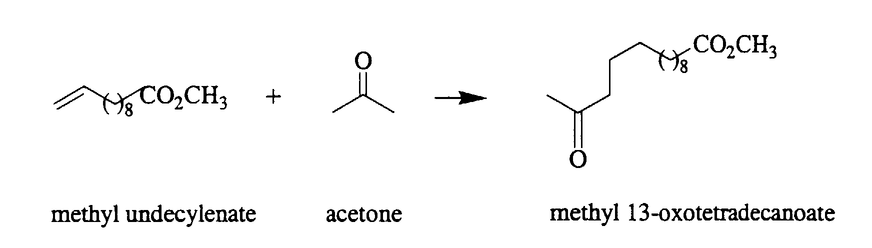

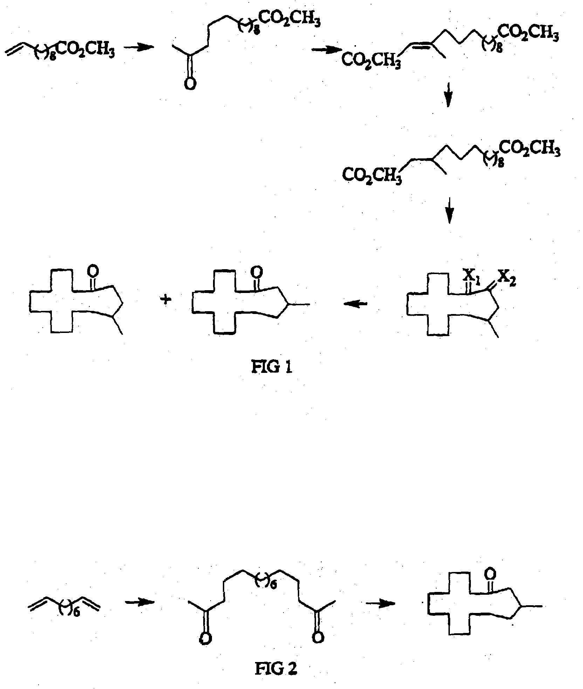

The resulting methyl 13-oxotetradecanoate can then be converted, eg using known techniques, to muscone. For example, in a subsequent 4 step conversion, Wittig-Homer (or Wadsworth-Emmons) reaction of methyl 13-oxotetradecanoate with trimethyl phosphonoacetate gives 3-methylpentadec-2-endioic acid dimethyl ester. Reduction of this material, by catalytic hydrogenation, gives dimethyl 3-methylpentadecanedioate. Cyclisation of this material, eg on treatment with sodium in refluxing xylene, under nitrogen, gives a mixture of acyloins, 2-hydroxy-4-methyl-1-cyclopentadecanone and 2-hydroxy-14-methyl-1-cyclopentadecanone, which are not readily separable. Heating the mixture of acyloins with zinc and hydrochloric acid produces a mixture of muscone and 4-methylcyclopentadecanone. This series of reactions is illustrated in Figure 1. Muscone can be separated from the resulting mixture, eg by silica gel chromatography. However, the mixture has good olfactive properties and can be used as a fragrance or perfume ingredient.

For reaction scheme 2, reaction of 1,9-decadiene (R₃=H, R₄=H, m=6) with acetone gives 2,15-hexadecanedione, as follows: 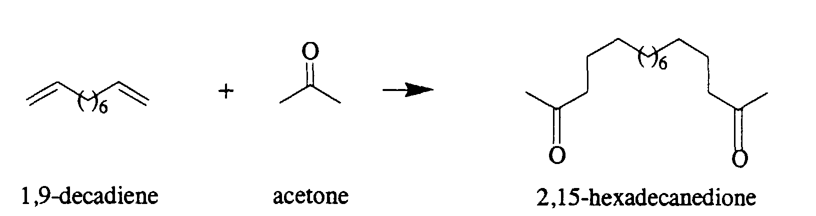 1,9-decadiene may be prepared in known manner, eg by ethenylation of cyclooctene (Szmant, H.H., *'Organic Building Blocks of the Chemical Industry',* Wiley Interscience, New York, p310 (1989)).

The resulting 2,15 hexadecanedione may be converted to muscone, eg in various known ways including by intramolecular aldol reaction (Stoll, M., Rouve, A. *Helv. Chim. Acta,* **30**, 2019, (1947), Tsuji. J., *et al., Tet. Lett.,* 2257 (1979), Tsuji, J., *et al., Bull. Chem. Soc. Jpn.,* 1417, **53** (1980) and Sakurai, H., *et al., J Organomet. Chem.,* **264**, 229, (1984)), followed by hydrogenation (see Figure 2). A further approach is high temperature cyclisation over a suitable catalyst, as described in EP 400509 of BASF.

In a preferred aspect the invention provides a method of preparing a macrocyclic ketone, particularly muscone, comprising radical addition of ketone to alkene in accordance with the invention.

The radical addition will usually be the first step of the method and may result in production of materials that can be converted to macrocyclic ketones in known manner, eg as described above.

The macrocyclic ketones may be produced separately or in the form of mixtures. Both separate ketones and mixtures may have fragrance properties that render the materials suitable for perfumery use in known manner.

The invention also covers macrocyclic ketones, particularly muscone, produced by the method of the invention, both as separate ketones and mixtures. In particular the invention covers muscone, alone and in admixture with 4-methylcyclopentadecanone, produced by the method of the invention.

The macrocyclic ketones may be used as ingredients of perfumes and perfumed products in known manner, eg as described in WO 98/09933.

In a further aspect the invention also covers a perfume or perfumed product comprising a macrocyclic ketone, particularly muscone, produced by the method of the invention, in an olfactively effective amount.

In perfumes an amount of 0.01% by weight or more of a macrocyclic ketone produced according to the invention will generally have a clearly perceptible olfactive effect. Preferably the amount is in the range 0.1 to 80% by weight, more preferably at least 1%. The amount of the ketone produced according to the invention present in products will generally be at least 10 ppm by weight, preferably at least 100 ppm, more preferably at least 1000 ppm. However, levels of up to about 20% by weight may be used in particular cases, depending on the product to be perfumed.

The invention will be further described, by way of illustration, in the following Examples and with reference to the accompanying Figures, in which:
Figure 1 shows the reaction scheme for radical addition of acetone to methyl undecylenate to produce methyl 13-oxotetradecanoate, and subsequent reaction of this to produce a mixture of muscone and 4-methylcyclopentadecanone; and
Figure 2 shows the reaction scheme for radical addition of acetone to 1,9-decadiene to produce 2,15-hexadecanedione, and subsequent reaction of this to produce muscone.

### Example 1

This concerns radical addition of acetone to methyl undecylenate, in accordance with reaction scheme 1, to produce methyl 13-oxotetradecanoate, and subsequent reaction of this to produce a mixture of muscone and 4-methylcyclopentadecanone. The reaction scheme is shown in Figure 1.

### Synthesis of Methyl 13-oxotetradecanoate

A mixture of potassium acetate (70g; 720mmol), manganese (II) acetate tetrahydrate (1g; 0.4mmol), acetone (300ml) and glacial acetic acid (200ml) was heated at 70°C under N₂. Methyl undecylenate (39g; 0.21 mol) was then added, followed by the addition of solid potassium permanganate (12.6g; 0.8 equivalents), which was added in very small portions at 70°C over a period of 4 hours.

The reaction mixture was cooled to room temperature, diluted with water (1.5L) and extracted into dichloromethane (2 x 500ml). The combined organic extracts were washed with saturated NaHCO₃ (aq), then water, and finally dried over anhydrous MgSO₄. The solvent was removed *in vacuo* to give a pale yellow oil (56.0g). glc [SE 54; 100-250°C@4°C min⁻¹] indicated a high degree of conversion.

| methyl undecylenate | crude produced |
|---|---|
| 12.354 min 99.1% | 25.680 min (81.5%) |

The crude product was vacuum distilled, giving a pale yellow oil which solidified 20.9g, 39%. Glc (as above) indicated 92% purity.
- ¹³C NMR (CDCl₃): 23.7514(CH₂), 24.8371(CH₂),
29.0197(CH₂), 29.0579(CH₂),
29.1191(CH₂), 29.2643(CH₂), 29.2796(CH₂),
29.3026(CH₂), 29.3790(CH₂), 29.7002(CH₃), 34.1146(CH₂),
43.8177(CH₂), 52.4324(OCH₃), 174.1612(C=O, ester),
209.1505(C=O).

### Synthesis of Dimethyl 3-Methyl-2-pentadecanedioate

A Wittig-Homer (or Wadsworth-Emmons) reaction was followed by catalytic hydrogeneration.

Trimethyl phosphonoacetate (82g; 0.45 mol) was added dropwise with stirring to a suspension of oil-free sodium hydride (12g; 0.5mol) in tetrahydrofuran (THF) (300ml). After 1 hour, methyl 13-oxotetradecanoate, [102.4g; 0.4mol], dissolved in THF (150ml) was added dropwise, and the reaction mixture refluxed, with stirring, under nitrogen for 3 hours. After cooling, the reaction mixture was poured into water and extracted into diethyl ether. The combined ether extracts were washed, and dried over MgSO₄. Glc [SE54; 100-250°C@4°C min⁻¹] indicated a 90% conversion.

The solvent was removed *in vacuo* and the residue was vacuum distilled, using a Vigreux column to give dimethyl (E-, Z-)-3-methyl-2-pentadecenedioate as a colourless oil (114.3g; 91.6%), bp 170°C at 0.15m bar.

This product was dissolved in methanol (400ml) and 5% palladium on carbon (5g) was added. The reaction mixture was hydrogenated in a 1 litre Buchi autoclave at 5 bar H₂ pressure for 7 hours. Glc (as above) now showed one major peak (90.4%). The catalyst was removed by filtration through Celite and the solvent removed to give a crude product which was vacuum distilled to give dimethyl 3-methyl-2-pentadecanedioate (104.5g).

### Synthesis of 2-Hydroxy-4-MethyI-1-Cyclopentadecanone and 2-Hydroxy-14-Methyl-1-Cyclopentadecanone

Dimethyl 3-methyl-2-pentadecandioate (11.3g; 0.036mol) was dissolved in xylene (80ml) and added, over one hour, to finely-dispersed sodium microgranules (<0.1mm particle size, Acros) in xylene (500ml). The reaction mixture was maintained under nitrogen throughout, and refluxing was continued for 30 minutes following complete addition.

The mixture was cooled, and treated, in a stream of nitrogen, with ethanol (25ml). The xylene solution was washed free of alkali, dried over MgSO₄, then the solvent was removed *in vacuo.* The residue was short-path distilled to give a pale green oil (8.7g; 93%) b.p. 125°C @ 0.1 mbar. glc [SE54; 100-250°C @ 4°C] showed only one product peak 28.043 min 91%. ¹H and ¹³C NMR indicated the presence of both acyloins, 2-hydroxy-4-methyl-1-cyclopentadecanone (X₁=O, X₂=H,OH in Figure 1) and 2-hydoxy-14-methyl-1-cyclopentadecanone (X₁ =H,OH, X₂=O in Figure 1).

The experiment was repeated (5x scale) in order to obtain further material for the final stage.

### Synthesis of Muscone and 4-Methylcyclopentadecanone

The mixture of acyloin products [39.3g; 0.155 mol] was dissolved in 1,4-dioxan (1 litre). Zinc pellets (60g) were added, and the reaction mixture was refluxed with stirring while passing HCl(g). After 2 hours, glc [SE54; 100-250°C @4°C min⁻1] indicated complete conversion to two products.

The reaction mixture was filtered to remove excess zinc, then washed with water, followed by NaHCO₃(aq), then a final water wash. The solvent was removed *in vacuo,* and the crude residue was chromatographed [silica; hexane 85% Et₂O 15%] to give a colourless oil, which was vacuum distilled using a short Vigreux side arm. This gave a colourless oil (27.3g; 74%), being a mixture of

| | |
|---|---|
| 3-methylcyclopentadecanone (31.2%) | Kovats 1870 |
| 4-methylcyclopentadecanone (65.7%) | Kovats 1888 |

The mixture has good olfactive properties and is useful as a fragrance material as it stands.

Muscone can be separated from the mixture if desired, eg by silica gel chromatography.

### Example 2

In a variant of Example 1, butan-2-one (2-butanone) was used in place of acetone and was added with high regioselectivity to methyl undecylenate. The product was homologated and cyclised to give a mixture of 3,4-dimethylcyclopentadecanone and 4,5-dimethylcyclopentadecanone in analogous manner to Example 1. The resulting mixture smelled musky.

### Synthesis of 3,4-Dimethylcyclopentadecanone and 4,5-Dimethylcyclopentadecanone

### a) Synthesis of Methyl 12-Methyl-13-oxotetradecanoate

A mixture of potassium acetate (140g), manganese (II) acetate tetrahydrate (2.0g), butan-2-one (500g), and glacial acetic acid (300ml) was heated @ 70°C under N₂. Methyl undecylenate (80g; 0.43mol) was added, followed by portionwise addition of potassium permanganate (25.0g) over 2 hours.

The reaction mixture was cooled to room temperature, water (2000mls) was added, and the reaction mixture was extracted into diethyl ether. The organic extracts were washed thoroughly with water, then aqueous sodium bicarbonate. Removal of solvent gave a pale yellow oil, which was subjected to vacuum distillation through a Vigreux column.

After a fore-run consisting of unreacted methyl undecylenate, the title material was obtained as a colourless oil (32.0g) bp 146°C @ 0.6mm Hg. M+270 ¹H and ¹³C NMR confirmed the structure, and indicated the absence of the isomeric methyl 13-oxopentadecanoate.
- ¹³C NMR (CDCl₃): 7.7732 (CH₃), 16.4670(CH₃), 24.9238(CH₂), 27.3171(CH₂),
29.0681(CH₂), 29.2210(CH₂), 29.3815(CH₂), 29.4810(CH₂),
29.6721(CH₂), 33.1435(CH₂), 34.0534(CH₂), 34.1917(CH₂),
46.0734(CH), 51.3569(OCH₃), 174.2095(C=O ester),
215.3541(C=O).

### b) Synthesis of Dimethyl 3,4-Dimethylpentadecanedioate

Trimethyl phosphonoacetate (25.0g; 0.133mol) was added dropwise with stirring to a suspension of oil-free sodium hydride (3.6g; 0.15mol) in dry THF (150ml). After complete addition, methyl 12-methyl-13-oxotetradecanoate (32.0g; 0.12mol) was added dropwise, and the reaction mixture was refluxed with stirring for a total of 8 hours.

Most THF was removed *in vacuo* and the residue was partitioned between diethyl ether and water. The organic layer was separated, washed with water, and dried over MgSO₄. Chromatography on silica, using a mixture of hexane (90%) and diethyl ether (10%) as eluent gave dimethyl 3,4-dimethyl-2-pentadecenedioate (31.2g) as a colourless oil.

This unsaturated diester (25.0g) was dissolved in methanol (250ml), and 5% Pd/C added. The reaction mixture was hydrogenated @ 6bar hydrogen pressure until uptake ceased. The catalyst was removed by filtration through Celite and the solvent removed *in vacuo* to give a colourless oil. Chromatography (conditions as above) gave a colourless oil (23.2g), identified as dimethyl 3,4-dimethylpentadecanedioate. Glc [SE54: 100°C-250°C@4°C/min] indicated 98% purity.

### c) Synthesis of 3,4-Dimethlylcyclopentadecanone and 4,5-Dimethylcylclopentadecanone

Dimethyl 3,4-dimethylpentadecanedioate (14.0g: 0.042mol) was dissolved in xylene (80ml), and added dropwise over 1 hour to a suspension of sodium (0,1mm particle size) in xylene (80ml). The addition was carried out under nitrogen, and the reaction mixture was refluxed for a further 30 minutes under nitrogen before cooling, and treating dropwise with ethyl alcohol (100ml.), in a stream of nitrogen. The xylene solution was then washed free of alkali with water.

TLC [silica:hexane 80%, diethyl ether 20%] indicated the reaction to have gone to completion. The xylene solution was dried over MgSO₄, and the solvent removed *in vacuo* to give the mixture of acyloin intermediates as a pale yellow oil (8.2g). This was dissolved in 1,4-dioxan (250ml), and zinc dust (15g) added. The reaction mixture was heated to reflux and hydrogen chloride gas was passed with stirring for 1 hour. The reaction mixture was filtered through Celite, and the solvent removed from the filtrate *in vacuo* to give a colourless oil (3.8g). This was chromatographed on silica, using hexane as eluent, gave a colourless oil (1.3g), identified as 1,2-dimethylcyclopentadecane, obtained by over-reduction. Further elution, using a mixture of hexane (90%) and methyl tert-butylether (10%) as eluent, gave a colourless oil (1.1g) identified as a mixture of 3,4-dimethylcyclopentadecanone and 4,5-dimethylcyclopentadecanone. The mixture had a musky smell.

| glc[SE54 100°C-250°C @ 4°C/min] | | |
|---|---|---|
| 26.052min | 33.6% | M+252 |
| 26.210min | 14.8% | M+252 |
| 26.500min | 33.1% | M+252 |
| 26.722min | 11.5% | M+252 |

### Example 3

This concerns radical addition of acetone to 1,9-decadiene, in accordance with reaction scheme 2, to produce 2,15-hexadecanedione. This may be subsequently reacted in known manner to produce muscone. The reaction scheme is shown in Figure 2.

### Synthesis of 2,15-Hexadecanedione

A mixture of potassium acetate (70g; 720mmol), manganese (II) acetate tetrahydrate (1g; 0.4mmol), acetone (300ml) and glacial acetic acid (150ml) was heated at 70°C under N₂. 1,9-Decadiene (35.0g; 0.25 mol) was then added, followed by solid potassium permanganate (12.6g; 0.8 equivalent), which was added in very small portions at 70°C over a period of 4 hours. The reaction mixture was then cooled, diluted with water (1500ml), and extracted into dichloromethane. The combined organic extracts were washed with saturated NaHCO₃ (aq), then water, and finally dried over MgSO₄. The solvent was removed *in vacuo* to give a pale yellow oil (58.2g). Glc [SE 54; 100-250°C@4°C min⁻¹] indicated 70.4% conversion.

Tlc [silica:hexane 50%, Et₂O 50%] indicated a major product, which was separated by chromatography (as Tlc), to give a colourless solid (25.2g). This was recrystallised to give colourless crystals (18.9g, 29%), mp 80°C (hexane).
- ¹³C NMR (CDCl₃):: δ209.2(C=O), 43.7 (CH₂C=O), 29.8 (CH₃C=O), 29.5 (CH₂)
29.4 (CH₂), 29.3 (CH₂), 29.1 (CH₂), 23.8 (CH₂).

The 2,15-hexadecanedione may be converted to muscone in known manner, eg by an internal Aldol reaction followed by hydrogenation, eg using 5% palladium on carbon catalyst.

A different, cheaper approach involves the high temperature cyclisation over a suitable catalyst. Such methodology is described in EP 400509 of BASF. Such catalyst methods enable greater than 60% conversion with high selectivity, providing a short and inexpensive synthesis of racemic muscone.

In a variant of Example 3, the procedure was repeated using a longer diene (m=8) (synthesised by Wittig reaction) in place of 1,9-decadiene (m=6). The expected product was obtained.

## Claims

1. Radical addition of ketone to alkene of the form: 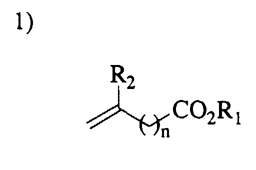 where
R₁ = alkyl
R₂ = H or alkyl
n = 1 to 8
or 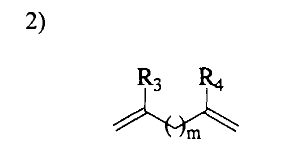 where
R₃ = H or lower alkyl
R₄ = H or lower alkyl
m = 2 to 8.

2. A process according to claim 1, wherein initiation of the addition reaction is carried out by *in situ* generation of manganese (III) from manganese (II) by redox reaction.

3. A process according to claim 1 or 2, wherein n=8 or m=5,6,7 or 8.

4. A process according to any one of the preceding claims, comprising reaction of methyl undecylenate with acetone to produce methyl 13-oxotetradecanoate.

5. A process according to claim 4, wherein the methyl 13-oxotetradecanoate is converted to muscone.

6. A process according to any one of claims 1 to 3, comprising reaction of 1,9-decadiene with acetone to produce 2,15-hexadecanedione.

7. A process according to claim 6, wherein the 2,15-hexadecanedione is converted to muscone.

8. A method of preparing a macrocyclic ketone, particularly muscone, comprising radical addition of ketone to alkene in accordance with any one of the preceding claims.

9. Macrocyclic ketones, particularly muscone, produced by the method of claim 8.

10. A perfume or perfumed product comprising a macrocyclic ketone, particularly muscone, produced by the method of claim 8.
